# EUROPEAN PATENT APPLICATION

(11) **EP 0 852 947 A2**
(43) Date of publication of application: **15.07.1998**
(21) Application number: 97121664.3
(22) Date of filing: 09.12.1997
(51) Int. Cl.: A61K 7/50

(54) **Detergent composition**

(30) Priority: 20.12.1996 JP 341229/96; 20.12.1996 JP 341230/96
(71) Applicant: Kao Corporation, Chuo-Ku Tokyo (JP)
(72) Inventor: Sonohara, Hiroshi, c/o Kao Corporation, Wakayama-shi, Wakayama (JP); Hasebe, Keiko, c/o Kao Corporation, Wakayama-shi, Wakayama (JP); Shonaka, Masafumi, c/o Kao Corporation, Wakayama-shi, Wakayama (JP); Doi, Yasuhiro, c/o Kao Corporation, Wakayama-shi, Wakayama (JP)
(74) Representative: Kindler, Matthias, Dr. Dipl.-Chem.

(57) **Abstract**

Disclosed herein is a detergent composition comprising (A) 5-95 wt.% of a higher fatty acid salt as a principal detergent base, (B) 0.2-5 wt.% of a cationic germicide, and (C) 0.1-10 wt.% of at least one selected from the group consisting of amphoteric surfactants, amine oxide type surfactants and amidoamino acid type surfactants. The detergent composition has excellent detergency and foamability, and a high germicidal effect because a cationic germicide can be stably incorporated therein.

## Description

### BACKGROUND OF THE INVENTION

### Field of the Invention:

The present invention relates to detergent compositions, and particularly to detergent compositions which have excellent detergency and foamability, and antipruritic and deodorant effects on the body because a cationic germicide can be stably incorporated therein to exhibit high germicidal and antibacterial effects, and are suitable for, in particular, skin detergents.

### Description of the Background Art:

Higher fatty acid salts as anionic surfactants have heretofore been often used as main base materials for detergents, in particular, skin detergents because they have excellent detergency and foamability.

On the other hand, many investigations have been made as to detergents having a germicidal effect, and detergent compositions making use of a cationic germicide as a germicide have been known. When such a cationic germicide is incorporated in a detergent composition comprising an anionic surfactant as a principal base material, however, the cationic germicide and the anionic surfactant form a complex, so that such problems that the germicidal effect cannot be sufficiently exhibited, and the foamability of the anionic surfactant is impaired have arisen.

As methods for preventing such reduction in germicidal activity, there have been used a method in which the cationic germicide is coated with a high molecular weight nonionic surfactant or a pH-sensitive polymer, and a method in which the cationic germicide is added in excess. However, the former polymer coating method reduces the activity of the cationic germicide. On the other hand, the method making use of the germicide in excess is not preferable from an economical point of view.

### SUMMARY OF THE INVENTION

Accordingly, it is an object of the present invention to provide detergent compositions which have excellent detergency and foamability, can last foams produced longer, have excellent antipruritic and deodorant effects on the body because a cationic germicide can be stably incorporated therein to bring about high germicidal and antibacterial effects, and are hence suitable for skin detergents.

In view of the foregoing circumstances, the present inventors have carried out an extensive investigation with a view toward solving the above-described problems. As a result, it has been found that when a higher fatty acid salt, a specific cationic germicide, and at least one selected from the group consisting of amphoteric surfactants, amine oxide type surfactants and amidoamino acid type surfactants are incorporated in combination in specific proportions, a detergent composition, which has high detergency and foamability, and satisfactory germicidal, antibacterial, antipruritic and deodorant effects on the skin, can be provided, thus leading to completion of the present invention.

According to the present invention, there is thus provided a detergent composition comprising the following components (A), (B) and (C):
(A) 5-95 wt.% of a higher fatty acid salt represented by the general formula (1):

   R¹COOM¹ (1)

   wherein R¹ is a linear or branched alkyl or alkenyl group having 7-21 carbon atoms, and M¹ is an alkali metal or alkaline earth metal atom, or an ammonium, alkylammonium or alkanolammonium group;
(B) 0.2-5 wt.% of at least one selected from the group consisting of cationic germicides represented by the following general formulae (2), (3), (4) and (5): 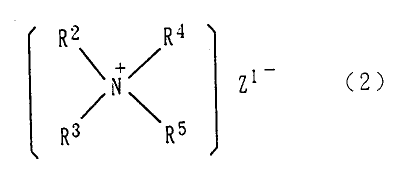 wherein R² and R³ may be the same or different from each other and are independently a long-chain alkyl, long-chain alkenyl or long-chain hydroxyalkyl group having 6-14 carbon atoms, said groups R² and R³ having 16-26 carbon atoms in total, R⁴ and R⁵ may be the same or different from each other and are independently an alkyl or hydroxyalkyl group having 1-3 carbon atoms, or a polyoxyethylene group having an average number of moles of at most 10, and Z¹ is a halogen atom, an anionic residue of an amino acid, a fatty acid, or a phosphate, phosphonate, sulfonate or sulfate having a linear or branched alkyl or alkenyl group having 1-30 carbon atoms, or an anionic oligomer or polymer having a styrenesulfonic acid having a polymerization degree of at least 3 or containing a condensate of a sulfonated polycyclic aromatic compound, which may have a hydrocarbon group as a substituent group, with formalin; 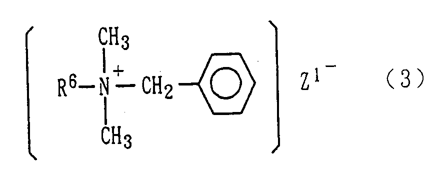 wherein R⁶ is a hydrocarbon group having 8-14 carbon atoms or a group represented by the formula: 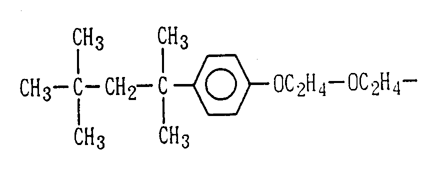 and Z¹ is a halogen atom, an anionic residue of an amino acid, a fatty acid, or a phosphate, phosphonate, sulfonate or sulfate having a linear or branched alkyl or alkenyl group having 1-30 carbon atoms, or an anionic oligomer or polymer having a styrenesulfonic acid having a polymerization degree of at least 3 or containing a condensate of a sulfonated polycyclic aromatic compound, which may have a hydrocarbon group as a substituent group, with formalin; 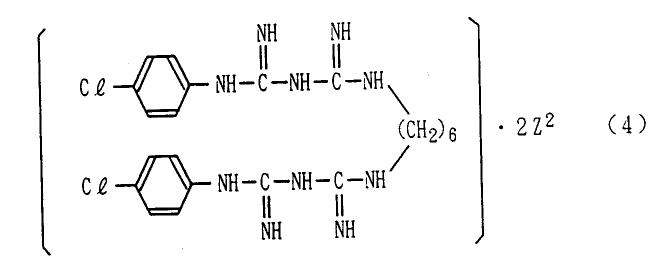 wherein Z² is gluconic acid, acetic acid or hydrochloric acid; and 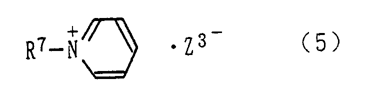 wherein R⁷ is a linear or branched alkyl group having 6-18 carbon atoms, Z³ is a halogen atom, an anionic residue of an amino acid, a fatty acid, or a phosphate, phosphonate, sulfonate or sulfate having a linear or branched alkyl or alkenyl group having 1-30 carbon atoms, or an anionic oligomer or polymer having a styrenesulfonic acid having a polymerization degree of at least 3 or containing a condensate of a sulfonated polycyclic aromatic compound, which may have a hydrocarbon group as a substituent group, with formalin, and polyhexamethylene biguanide; and
(C) 0.1-10 wt.% of at least one selected from the group consisting of amphoteric surfactants, amine oxide type surfactants and amidoamino acid type surfactants, wherein the higher fatty acid salt of component (A) is contained as a principal detergent base.

According to the present invention, there is also provided a detergent composition further comprising, as a component (D) in addition to the components (A) to (C), a higher fatty acid represented by the general formula (6):

R¹⁸-COOH (6)

wherein R¹⁸ is a linear or branched alkyl or alkenyl group having 7-21 carbon atoms, in an amount 0.01-1 time as much as the weight of the component (A).

According to the present invention, there is further provided a detergent composition further comprising, as a component (E) in addition to the components (A) to (C) or (A) to (D), at least one metal chelating agent.

The detergent compositions according to the present invention have excellent detergency and foamability, and sufficient antipruritic and deodorant effects on the body because a cationic germicide can be stably incorporated therein to exhibit high germicidal and antibacterial effects and broad germicidal and antibacterial spectra.

The above and other objects, features and advantages of the present invention will be readily appreciated as the same becomes better understood from the preferred embodiments of the present invention, which will be described subsequently in detail, and from the appended claims.

### DETAILED DESCRIPTION OF THE PREFERRED EMBODIMENTS

Specific examples of the higher fatty acid salt of the component (A) useful in the practice of the present invention include alkali metal salts, alkaline earth metal salts, ammonium salts, alkylammonium salts and alkanolamine salts of caprylic acid, capric acid, undecanoic acid, lauric acid, tridecanoic acid, myristic acid, pentadecanoic acid, palmitic acid, heptadecanoic acid, stearic acid, oleic acid, elaidic acid, linoleic acid, linolenic acid, arachidic acid, and fatty acids derived from animal and vegetable fats and oils, such as coconut oil fatty acid and beef tallow fatty acid containing these fatty acids. Preferable examples thereof include those in which R¹ in the general formula (1) is a linear or branched alkyl or alkenyl group having 11-17 carbon atoms.

It is preferable that M¹ in the general formula (1) be suitably varied according to the form of the detergent composition according to the present invention. Specifically, when the detergent composition is used in the form of, for example, liquid or paste, M¹ is preferably a potassium atom, or an ammonium or triethanolammonium group. When the composition is used in the form of solid, M¹ is preferably a sodium atom.

The higher fatty acid salts of the component (A) are incorporated as a principal detergent base for the detergent compositions according to the present invention, and may be used either singly or in any combination thereof in the detergent compositions. The component (A) is incorporated in a proportion of at least 50 wt.% (hereinafter indicated merely by "%") based on the total weight of the detergent surfactants.

The higher fatty acid salt of the component (A) is preferably incorporated in a varied amount according to the form of the detergent composition according to the present invention. Specifically, when the detergent composition is provided in the form of, for example, liquid, the component (A) is preferably incorporated in a proportion of 5-50%, particularly, 10-40% based on the total weight of the composition. When the composition is provided in the form of paste, the component (A) is preferably incorporated in a proportion of 10-80%, particularly, 15-40% based on the total weight of the composition. When the composition is provided in the form of solid, the component (A) is preferably incorporated in a proportion of generally 60-95%, particularly, 70-95% based on the total weight of the composition.

The cationic germicide of the component (B) useful in the practice of the present invention is at least one selected from the group consisting of quaternary ammonium salts represented by the general formula (2), benzalkonium salts or benzethonium salts represented by the general formula (3), chlorhexidine salts represented by the general formula (4), pyridinium salts represented by the general formula (5) and polyhexamethylene biguanide.

Here, polyhexamethylene biguanide corresponds to that described as POLYAMINOPROPYL BIGUANIDE in International Cosmetic Ingredient Dictionary, the fifth edition, of CTFA, and is known by the trade mark of "Cosmocil" or "Mikrokill".

In the general formulae (2) and (3), Z¹ is particularly preferably a halogen atom.

Specific preferable examples of the cationic germicide of the component (B) include benzalkonium chloride, benzethonium chloride, cetyl pyridinium chloride, chlorhexidine gluconate, chlorhexidine acetate and chlorhexidine hydrochloride. Of these, benzalkonium chloride and benzethonium chloride are particularly preferred. Further, benzalkonium chloride represented by the general formula (3a); 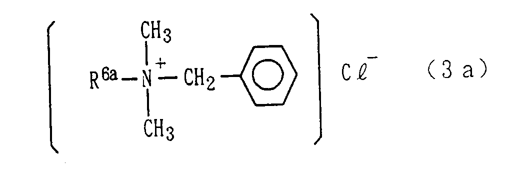 wherein R^{6a} is a linear or branched alkyl or alkenyl group having 8-14 carbon atoms, is preferred.

The cationic germicides of the component (B) may be used either singly or in any combination thereof, and are incorporated in a proportion of 0.2-5%, preferably 0.3-4%, particularly preferably 0.5-2% based on the total weight of the composition from the viewpoints of germicidal and antibacterial effects and irritativeness.

Examples of the amphoteric surfactants of the component (C) useful in the practice of the present invention include betaine type amphoteric surfactants represented by the following general formula (7): 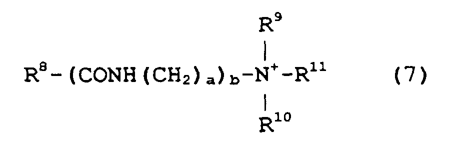 wherein R⁸ is a linear or branched alkyl or alkenyl group having 7-22 carbon atoms, a is an integer of 1-4, b is a number of 0 or 1, R⁹ and R¹⁰ are independently an alkyl group having 1-4 carbon atoms or -(CH₂CH₂O)_{d}H, in which d is a number of 1-3 on the average, and R¹¹ is -CH₂CH(OH)CH₂SO₃⁻, -(CH₂)ₑSO₃⁻ or -(CH₂)_{f}COO⁻, in which e is an integer of 2-5, and f is an integer of 1-3.

Of these, preferred are carbobetaine type surfactants represented by the general formula (7a): 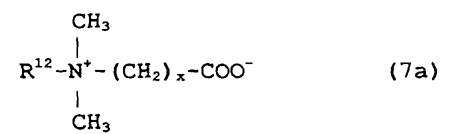 wherein R¹² is a linear or branched alkyl or alkenyl group having 8-20 carbon atoms or a group represented by R¹³CONH(CH₂)_{y}-, in which R¹³ is a linear or branched alkyl or alkenyl group having 7-19 carbon atoms, and y is an integer of 1-5, and x is an integer of 1-5; and sulfobetaine or hydroxysulfobetaine type surfactants represented by the general formula (7b) or (7c): 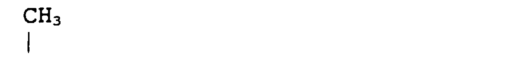 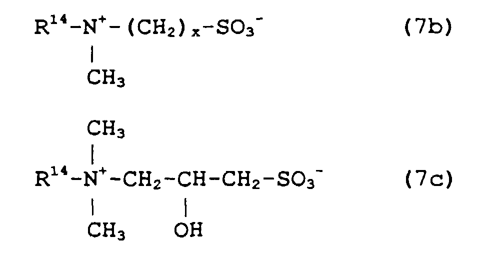 wherein R¹⁴ is a linear or branched alkyl or alkenyl group having 8-20 carbon atoms, and x has the same meaning as defined above. In the general formula (7a), R¹² is preferably a linear or branched alkyl or alkenyl group having 8-16 carbon atoms or R¹³CONH(CH₂)₃-, in which R¹³ is a linear or branched alkyl or alkenyl group having 7-15 carbon atoms, and x is preferably 1. In the general formula (7b) or (7c), R¹⁴ is preferably a linear or branched alkyl or alkenyl group having 8-16 carbon atoms. Further, fatty acid amide propylbetaines, wherein R¹² in the general formula (7a) is R¹³CONH(CH₂)_{y}-, in which R¹³ is a linear or branched alkyl or alkenyl group having 7-15 carbon atoms, and y is 3, are particularly preferred.

Examples of the amine oxide type surfactants of the component (C) useful in the practice of the present invention include those represented by the general formula (8): 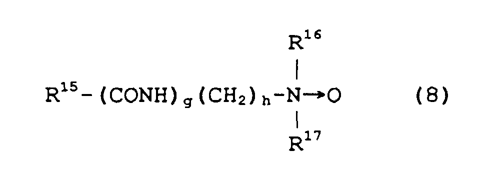 wherein R¹⁵ is a linear or branched alkyl or alkenyl group having 7-18 carbon atoms, R¹⁶ and R¹⁷ are independently a methyl, ethyl or hydroxyethyl group, g is 0 or 1, and h is an integer of 0-3. Of these, those represented by the following general formula (8a): 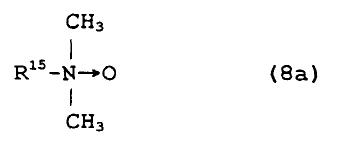 wherein R¹⁵ has the same meaning as defined above, are preferred.

Examples of the amidoamino acid type surfactants of the component (C) useful in the practice of the present invention include those represented by the general formula (9) or (10): 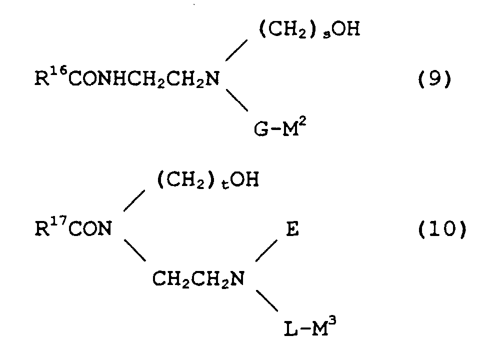 wherein R¹⁶ and R¹⁷ are independently a linear or branched alkyl or alkenyl group having 7-19 carbon atoms, s and t are independently an integer of 2-4, G and L are independently -CH₂CH(OH)CH₂SO₃, -(CH₂)ᵤSO₃ or -(CH₂)ᵥCOO, in which u is an integer of 2-5, and v is an integer of 1-3, E is a hydrogen atom or -L-M³, and M² and M³ are independently a hydrogen atom, an alkali metal or alkaline earth metal atom, or an ammonium, organic ammonium or a mixture thereof.

Specific examples of M² and M³ include hydrogen, lithium, sodium, potassium, 1/2 calcium, 1/2 magnesium, ammonium, monoethanolammonium, diethanolammonium, triethanolammonium, monoisopropanolammonium, diisopropanolammonium and triisopropanolammonium.

The amidoamino acid type surfactants are surfactants obtained, for example, by reacting 1 mol of an imidazoline derivative with 1-2 mol of monochloroacetic acid or an acrylic acid, have once called imidazoline type amphoteric surfactants or imidazolinium betaines, correspond to those described as SODIUM COCOAMPHOACETATE, SODIUM COCOAMPHOPROPIONATE, SODIUM COCOAMPHOHYDROXYPROPYLSULFONATE, SODIUM LAUROAMPHOACETATE and DISODIUM COCOAMPHODIACETATE in the CTFA's Dictionary, the fifth edition, and are known by the trade mark of "Miranol".

Said at least one surfactant of the component (C) selected from the group consisting of the amphoteric surfactants, amine oxide type surfactants and amidoamino acid type surfactants is incorporated in a proportion of 0.1-10%, preferably 1-8%, particularly preferably 2-7% based on the total weight of the composition from the viewpoints of detergency, foamability, and germicidal and antibacterial effects.

The weight ratio of the component (A) to the component (C) in the detergent compositions according to the present invention is preferably 1:1 to 50:1, more preferably 1:1 to 20:1, most preferably 2:1 to 15:1 from the viewpoint of the germicidal effect.

When a higher fatty acid represented by the general formula (6):

R¹⁸-COOH (6)

wherein R¹⁸ is a linear or branched alkyl or alkenyl group having 7-21 carbon atoms, is incorporated as a component (D) in addition to the above-described components in the detergent composition according to the present invention, the feel of foams produced therefrom is more improved.

The higher fatty acids of the component (D) may be used either singly or in any combination thereof. Specific examples thereof include caprylic acid, capric acid, undecanoic acid, lauric acid, tridecanoic acid, myristic acid, pentadecanoic acid, palmitic acid, heptadecanoic acid, stearic acid, oleic acid, elaidic acid, linoleic acid, linolenic acid, arachidic acid, and fatty acids derived from animal and vegetable fats and oils, such as coconut oil fatty acid and beef tallow fatty acid containing these fatty acids. Preferable examples thereof include those in which R¹⁸ in the general formula (6) is a linear or branched alkyl or alkenyl group having 11-17 carbon atoms.

In the detergent composition according to the present invention, the weight ratio of the higher fatty acid of the component (D) to the higher fatty acid salt of the component (A) is within a range of from 0.01:1 to 1:1, preferably from 0.05:1 to 0.5:1. The weight ratio, (D)/(A) of (D) to (A) may be suitably controlled by changing the compounding ratio of the component (A) to the component (D) or the neutralization degree of the component (D) in the detergent composition.

In the detergent compositions according to the present invention, a metal chelating agent may be incorporated as a component (E) to more enhance the germicidal and antibacterial effects of the compositions. No particular limitation is imposed on such a metal chelating agent of the component (E) so far as it has a capacity to chelate metal ions. However, examples thereof include aminopolycarboxylic acid type chelating agents, aromatic and aliphatic carboxylic acid type chelating agents, amino acid type chelating agents, ether polycarboxylic acid type chelating agents, phosphonic acid type chelating agents such as iminodimethylphosphonic acid (IDP), alkyldiphosphonic acids (ADPAs) and 1-hydroxyethane-1,1-diphosphonic acid (DEQUEST™ 2010), hydroxycarboxylic acid type chelating agents, phosphoric acid type chelating agents, chelating agents of the polyelectrolyte (including oligomer electrolyte) type, and dimethylglyoxime (DG). Each of these chelating agents may be in the form of a free acid or a salt such as the sodium, potassium or ammonium salt. The chelating agent may also be in the form of a hydrolyzable ester derivative.

Specific examples of the aminopolycarboxylic acid type chelating agents include:
a) compounds represented by the chemical formula, R²²(Y)₂;
b) compounds represented by the chemical formula, N(Y)₃;
c) compounds represented by the chemical formula, R²²-N(Y)-CH₂CH₂-N(Y)-R²²;
d) compounds represented by the chemical formula, R²²-N(Y)-CH₂CH₂-N(Y)₂;
e) compounds represented by the chemical formula, (Y)₂N-R²³-N(Y)₂; and
f) compounds similar to the compounds of e), which contain Y more than 4 groups, for example, a compound represented by the formula:

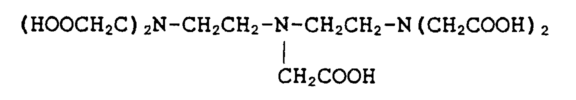

In the above formulae, Y is -CH₂COOH or -CH₂CH₂COOH, R²² is a group making up a known chelating agent, such as a hydrogen atom, or an alkyl, hydroxyl or hydroxyalkyl group, and R²³ is a group making up a known chelating agent of this kind, such as an alkylene or cycloalkylene group.

Typical examples of the aminopolycarboxylic acid type chelating agents include ethylenediaminetetraacetic acid (EDTA), cyclohexanediaminetetraacetic acid (CDTA), nitrilotriacetic acid (NTA), iminodiacetic acid (IDA), N-(2-hydroxyethyl)iminodiacetic acid (HIMDA), diethylenetriaminepentaacetic acid (DTPA), N-(2-hydroxyethyl)ethylenediaminetriacetic acid (EDTA-OH) and glycol ether diaminetetraacetic acid (GEDTA) as well as salts thereof.

Examples of the aromatic and aliphatic carboxylic acid type chelating agents used in the present invention include oxalic acid, malonic acid, succinic acid, glutaric acid, adipic acid, itaconic acid, aconitic acid, pyruvic acid, salicylic acid, acetylsalicylic acid, hydroxybenzoic acid, aminobenzoic acid (including anthranilic acid), phthalic acid, trimellitic acid and gallic acid as well as salts, methyl esters and ethyl esters thereof. Examples of the amino acid type chelating agents used in the present invention include glycine, serine, alanine, lysine, cystine, cysteine, ethionine, tyrosine, methionine, and salts and derivatives thereof.

Examples of the ether polycarboxylic acid type chelating agents used in the present invention include diglycolic acid, compounds represented by the following formula: 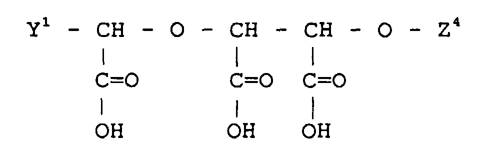 wherein Y¹ is a hydrogen atom, -CH₂COOH or -COOH, and Z⁴ is a hydrogen atom, -CH₂COOH or 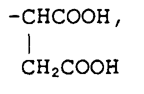 compounds similar to such compounds, and salts thereof (for example, the sodium salts).

Examples of the hydroxycarboxylic acid type chelating agents used in the present invention include malic acid, citric acid, glycolic acid, gluconic acid, heptonic acid, tartaric acid, lactic acid, and salts thereof. Examples of the phosphoric acid type chelating agents used in the present invention include orthophosphoric acid, pyrophosphoric acid, triphosphoric acid and polyphosphoric acid. Examples of the chelating agents of the polyelectrolyte (including oligomer electrolyte) type used in the present invention include acrylic acid polymers, maleic anhydride polymers, α-hydroxyacrylic acid polymers, itaconic acid polymers, copolymers composed of at least two monomers of these polymers, and epoxysuccinic acid polymers. Further, ascorbic acid, thioglycolic acid, phytic acid, glyoxylic acid and glyoxalic acid as well as salts thereof may also be suitably used as the chelating agents in the present invention.

Particularly preferable examples of the chelating agents include ethylenediaminetetraacetic acid (EDTA), succinic acid, salicylic acid, oxalic acid, lactic acid, fumaric acid, tartaric acid, 1-hydroxyethane-1,1-diphosphonic acid and salts thereof.

The metal chelating agent of the component (E) is incorporated in a proportion of 0.1-10%, preferably 0.2-5% based on the total weight of the composition.

In the detergent compositions according to the present invention, anionic surfactants commonly used in the classical detergent compositions may be incorporated so far as no detrimental influence is thereby imposed on the effects of the present invention. Examples of such anionic surfactants include alkylsulfates, alkyl ether sulfates, acylated isethionates, alkylsulfonates, sulfosuccinates, α-sulfo fatty acid esters, α-olefin sulfonates, phosphoric monoester type surfactants, alkyl ether carboxylates and acylated amino acids.

Besides, alkanolamide type surfactants may be further used in the present invention with a view toward improving foaming and the feel of foams to be produced. Examples of such alkanolamide type surfactants include those represented by the general formula (11): 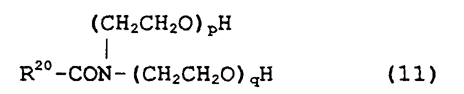 wherein R²⁰ is a linear or branched alkyl or alkenyl group having 7-19 carbon atoms, and p and q are independently an integer of 0-10, with the proviso that at least one of p and q is 1 or greater. Of these, those, in which R²⁰ in the general formula (11) is a linear or branched alkyl or alkenyl group having 7-15 carbon atoms, and p + q is 1-5, particularly 1 or 2, are preferred. The alkanolamide type surfactants are preferably incorporated in a proportion of 0.1-10%, more preferably 1-8%, most preferably 2-7% based on the total weight of the composition.

Further, silicone derivatives can be incorporated in a proportion of 0.1-5% in the detergent compositions according to the present invention, thereby giving users a good feeling of sliding or dryness. No particular limitation is imposed on the silicone derivatives so far as they are those commonly used in the classical detergents and cosmetics. Examples thereof include dimethyl polysiloxane, methylphenyl polysiloxane, polyether-modified silicones, epoxy-modified silicones, alkoxy-modified silicones, amino-modified silicones, fatty acid-modified silicones and fluorine-modified silicones.

In the detergent compositions according to the present invention, ingredients commonly incorporated in the classical cosmetics, drugs, food and the like, for example, germicides other than the cationic germicides, antiphlogistics such as glycyrrhetinic acid, dihydrocholesterin and allantoin, medicinally-effective agents and antiseptics; moisturizers such as propylene glycol, glycerol, diethylene glycol monoethyl ether, sorbitol and panthenol; colorants such as dyes and pigments; pearl-like-hue-imparting agents; chitosan derivatives such as hydroxypropylchitosan; various kinds of perfume bases; and besides ingredients described in ENCYCLOPEDIA OF SHAMPOO INGREDIENTS (MICELLE PRESS, 1985), may be incorporated in addition to the above-described components, as needed, so far as no detrimental influence is thereby imposed on the effects of the present invention.

The detergent compositions according to the present invention can be prepared in accordance with a method known *per se* in the art into various forms such as paste, gel, liquid and solid. The detergent compositions are suitable for use as detergents for the skin, particularly, the hands and body.

The present invention will hereinafter be described in more detail by the following examples. However, the present invention is not limited to these examples. Incidentally, the amounts of individual components to be incorporated in the following examples were expressed on the basis of active ingredients.

### Examples 1 to 6 and Comparative Examples 1 to 3:

Detergent compositions of their corresponding formulations shown in Table 1 were prepared in a method known *per se* in the art to evaluate them as to detergency, foamability, deodorant effect and antibacterial activity. The results are shown in Table 1.

### 〈Evaluation methods〉

### (1) Detergency:

Panelists composed of five men and five women were got to cleanse their bodies with each of the detergent compositions shown in Table 1, thereby organoleptically evaluating the composition as to the detergency in accordance with the following standard.

### (Evaluation standard)

- 5:: Felt that detergency was good;
- 4:: Felt that detergency was somewhat good;
- 3:: Felt that detergency was fair;
- 2:: Felt that detergency was somewhat poor; and
- 1:: Felt that detergency was poor.

The evaluation results as to the detergency were expressed in terms of the average value of the ten panelists in accordance with the following standard:
- ⓞ:: Average value of at least 4.0 (good);
- ○:: Average value of 3.2-3.9 (somewhat good);
- △:: Average value of 2.5-3.1 (fair); and
- X:: Average value of at most 2.4 (poor).

### (2) Foamability (foaming):

Panelists composed of five men and five women were got to cleanse their bodies with each of the detergent compositions shown in Table 1, thereby organoleptically evaluating the composition as to the foamability in accordance with the following standard.

### (Evaluation standard)

- 4:: Felt that foaming was somewhat good;
- 3:: Felt that foaming was fair;
- 2:: Felt that foaming was somewhat poor; and
- 1:: Felt that foaming was poor.

The evaluation results as to the foaming were expressed in terms of the average value of the ten panelists in accordance with the following standard::
- ⓞ:: Average value of 4.0 (good);
- ○:: Average value of 3.2-3.9 (somewhat good);
- △:: Average value of 2.5-3.1 (fair); and
- X:: Average value of at most 2.4 (poor).

### (3) Deodorant effect:

With respect to each of the detergent compositions shown in Table 1, a service test was conducted once a day for 2 weeks with panelists composed of five men and five women. The deodorant effect was evaluated by an expert valuer as to whether the panelists had a body smell or not upon elapsed time of 24 hours after the final cleansing, and ranked in accordance with the following standard.

### (Evaluation standard)

- 5:: Not smelled;
- 4:: Slightly smelled;
- 3:: Smelled;
- 2:: Considerably smelled; and
- 1:: Strongly smelled.

The evaluation results as to the deodorant effect were expressed in terms of the average value of the ten panelists in accordance with the following standard:
- ⓞ:: Average value of at least 4.0 (good);
- ○:: Average value of 3.2-3.9 (somewhat good);
- △:: Average value of 2.5-3.1 (fair); and
- X:: Average value of at most 2.4 (poor).

### (4) Antibacterial activity:

### Method of antibacterial test:

Germs, *Staphylococcus aureus* IFO 12732 (St. aureus) and *Escherichia coli* IFO 3972 (E. coli) to be tested were separately precultured at 35°C using an SCD medium (product of Nippon Seiyaku K.K.).

Each of the culture solutions of St. aureus and E. coli precultured was spread on an SCD agar medium (product of Nippon Seiyaku K.K.). After 50 µl of each of the detergent compositions shown in Table 1 were poured into the agar medium, a paper disk (diameter: 8 mm) washed with water was placed on the agar medium, on which the culture solution had been spread, to measure a diameter (mm) of an inhibitory circle after 24 hours at 35°C, thereby judging the antibacterial activity.

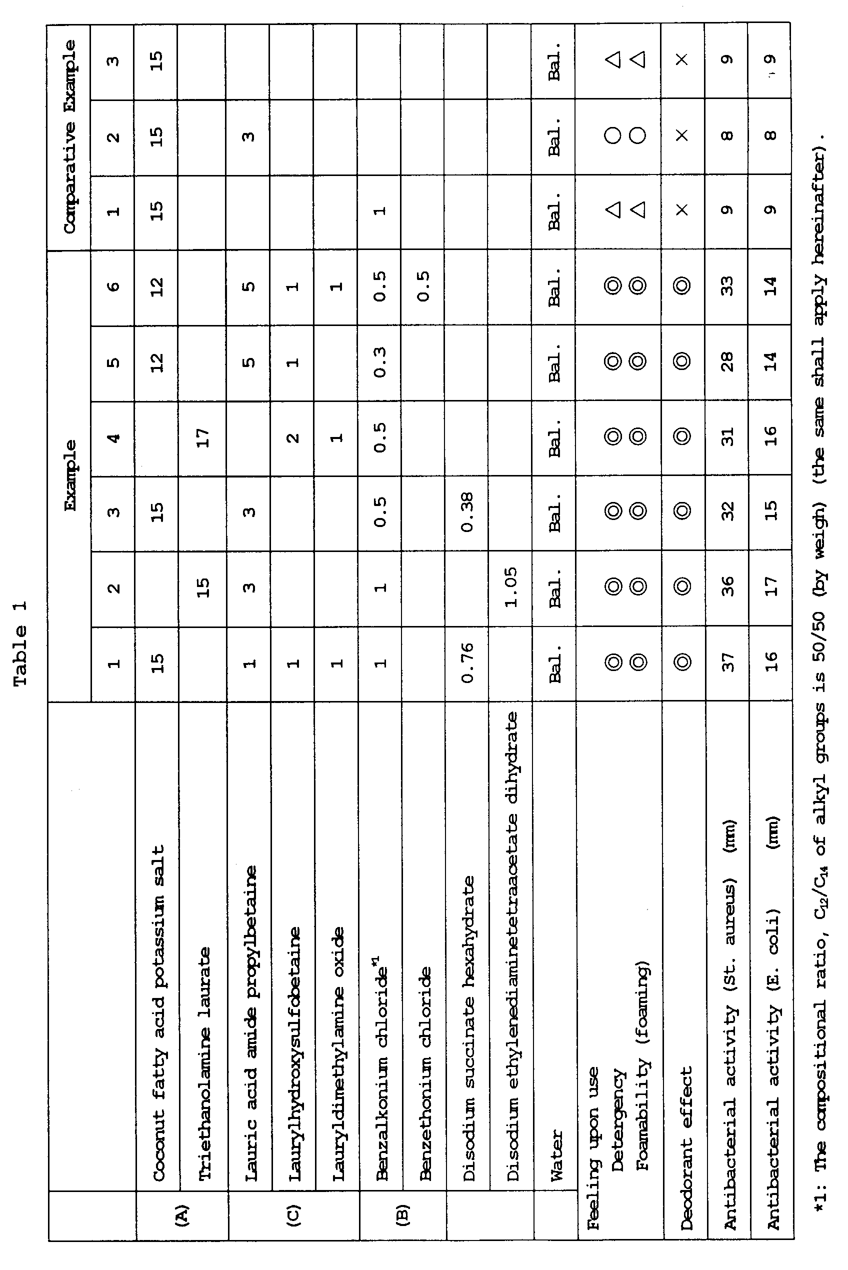

### Example 7:

A hand soap of a formulation shown below was produced in accordance with a method known *per se* in the art.

| | |
|---|---|
| Coconut fatty acid potassium salt | 16% |
| Lauric acid amide propylbetaine | 3% |
| Benzalkonium chloride^{*1} | 1% |
| Disodium ethylenediaminetetraacetate | 1% |
| Glycerol | 1% |
| Methylparaben | 0.2% |
| Perfume base | 0.1% |
| Water | Balance |
| Total | 100.0% |

| | |
|---|---|
| *1: The compositional ratio, C₁₂/C₁₄ of alkyl groups is 50/50 (by weigh) (the same shall apply hereinafter). | |

The hand soap thus obtained had excellent detergency and foamability, and a germicidal effect.

### Example 8:

A body shampoo of a formulation shown below was produced in accordance with a method known *per se* in the art.

| | |
|---|---|
| Coconut fatty acid potassium salt | 30% |
| Lauric acid amide propylbetaine | 2% |
| Lauryldimethylamine oxide | 0.5% |
| SODIUM COCOAMPHOACETATE | 0.5% |
| Benzalkonium chloride^{*1} | 1% |
| Disodium succinate | 0.8% |
| Glycerol | 1% |
| Magnesium stearate | 0.5% |
| Pearl-like-hue-imparting agent (ethylene glycol distearate) | 1% |
| Methylparaben | 0.2% |
| Perfume base | 0.2% |
| Water | Balance |
| Total | 100.0% |

| | |
|---|---|
| *1: The compositional ratio, C₁₂/C₁₄ of alkyl groups is 50/50 (by weigh) (the same shall apply hereinafter). | |

The body shampoo thus obtained had excellent detergency and foamability, and a deodorant effect.

### Example 9:

A solid body soap of a formulation shown below was produced in accordance with a method known *per se* in the art.

| | |
|---|---|
| Soap base^{*2} | Balance |
| Lauric acid amide propylbetaine | 2.0% |
| Benzethonium chloride | 0.7% |
| Chlorhexidine gluconate | 0.5% |
| Disodium ethylenediaminetetraacetate | 1% |
| 1-Hydroxyethane-1,1-diphosphonic acid solution (60% aqueous solution) | 0.4% |
| Dibutylhydroxytoluene | 0.1% |
| Magnesium stearate | 0.5% |
| Sodium benzoate | 0.5% |
| Methylparaben | 0.2% |
| Titanium oxide | 0.2% |
| Perfume base | 0.5% |
| Total | 100.0% |

| | |
|---|---|
| *2: Sodium salt of a mixed fatty acid composed of beef tallow/coconut oil = 80/20 (by weight); water content: 13%. | |

The solid body soap thus obtained had excellent detergency and foamability, and a high deodorant effect.

### Examples 10 to 15 and Comparative Examples 4 and 5:

Detergent compositions of their corresponding formulations shown in Table 2 were prepared in a method known *per se* in the art to evaluate them as to detergency, foamability, feel of foams, deodorant effect and antibacterial activity. The results are shown in Table 2.

Incidentally, with respect to each of the detergent compositions shown in Table 2, its evaluation was conducted in the same manner as described above except for the evaluation as to the feel of foams, which will be described subsequently.

### 〈Feel of foams〉

Panelists composed of five men and five women were got to cleanse their bodies with each of the detergent compositions shown in Table 2, thereby organoleptically evaluating the composition as to the feel of foams produced in accordance with the following standard.

### (Evaluation standard)

- 5:: Felt foams to be pleasant to the touch;
- 4:: Felt foams to be somewhat pleasant to the touch;
- 3:: Felt foams to be a toss-up between pleasant and unpleasant to the touch;
- 2:: Felt foams to be somewhat unpleasant to the touch; and
- 1:: Felt foams to be unpleasant to the touch.

The evaluation results as to the feel of foams were expressed in terms of the average value of the ten panelists in accordance with the following standard::
- ⓞ:: Average value greater than 4.0 (good);
- ○:: Average value of 3.6-4.0 (somewhat good);
- △:: Average value of 2.5-3.5 (fair); and
- X:: Average value of at most 2.4 (poor).

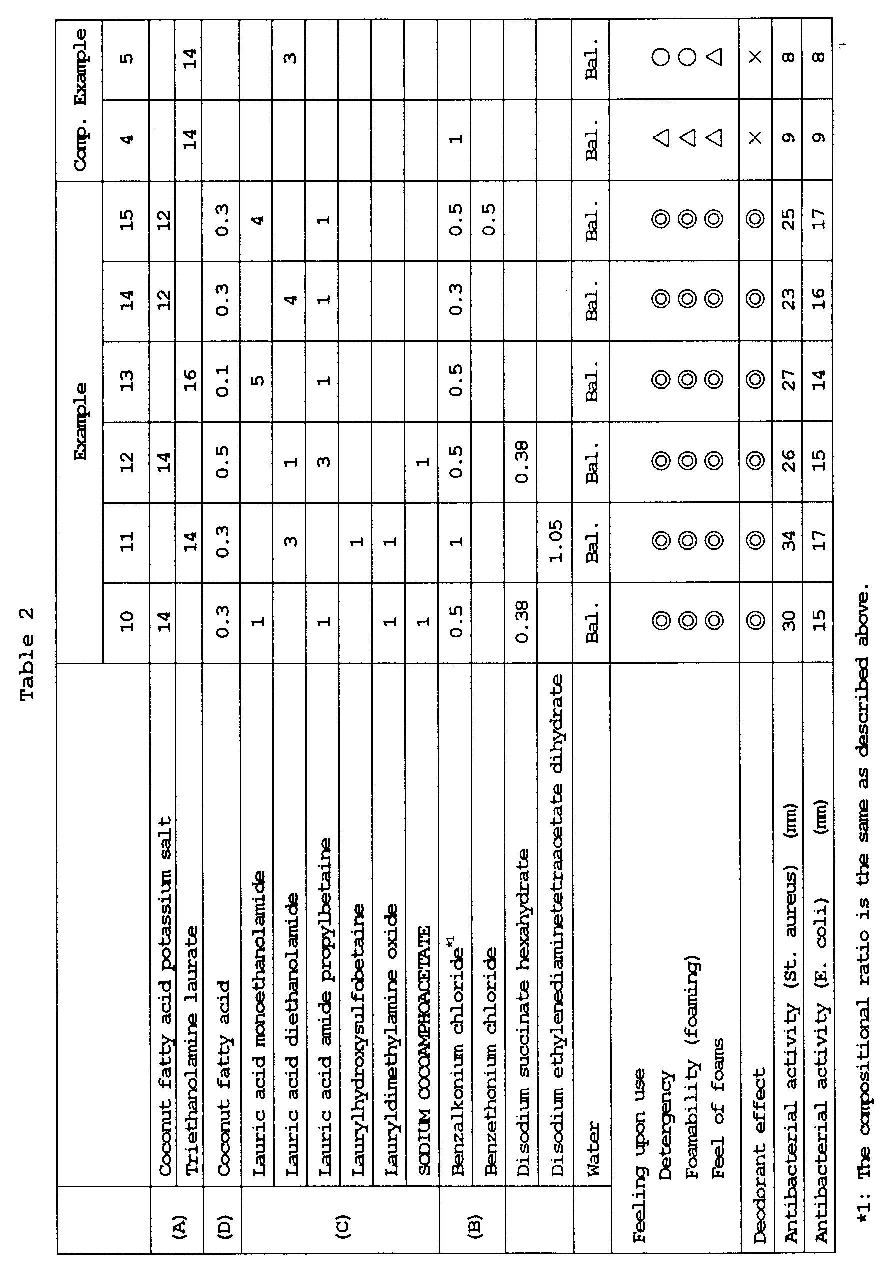

### Example 16:

A hand soap of a formulation shown below was produced in accordance with a method known *per se* in the art.

| | |
|---|---|
| Coconut fatty acid potassium salt | 16% |
| Coconut fatty acid | 5% |
| Lauric acid amide propylbetaine | 3% |
| Lauric acid diethanolamide | 1% |
| Benzalkonium chloride^{*1} | 0.5% |
| Disodium ethylenediaminetetraacetate | 0.6% |
| Perfume base | 0.1% |
| Water | Balance |
| Total | 100.0% |

| | |
|---|---|
| *1: The compositional ratio is the same as described above. | |

The hand soap thus obtained had excellent detergency and foamability, produced foams pleasant to the touch, and had germicidal power.

### Example 17:

A body shampoo of a formulation shown below was produced in accordance with a method known *per se* in the art.

| | |
|---|---|
| Coconut fatty acid potassium salt | 30% |
| Coconut fatty acid | 7% |
| Lauric acid amide propylbetaine | 2% |
| Lauryldimethylamine oxide | 0.5% |
| SODIUM COCOAMPHOACETATE | 0.5% |
| Benzalkonium chloride^{*1} | 1% |
| Disodium succinate | 0.8% |
| Glycerol | 1% |
| Magnesium stearate | 0.5% |
| Pearl-like-hue-imparting agent (ethylene glycol distearate) | 1% |
| Methylparaben | 0.2% |
| Perfume base | 0.2% |
| Water | Balance |
| Total | 100.0% |

| | |
|---|---|
| *1: The compositional ratio is the same as described above. | |

The body shampoo thus obtained had excellent detergency and foamability, produced foams pleasant to the touch, and had a deodorant effect.

### Example 18:

A solid body soap of a formulation shown below was produced in accordance with a method known *per se* in the art.

| | |
|---|---|
| Soap base^{*2} | Balance |
| Coconut fatty acid | 2% |
| Lauric acid amide propylbetaine | 2.0% |
| Benzethonium chloride | 1% |
| Disodium ethylenediaminetetraacetate | 1% |
| 1-Hydroxyethane-1,1-diphosphonic acid solution (60% aqueous solution) | 0.4% |
| Dibutylhydroxytoluene | 0.1% |
| Magnesium stearate | 0.5% |
| Sodium benzoate | 0.5% |
| Methylparaben | 0.2% |
| Titanium oxide | 0.2% |
| Perfume base | 0.5% |
| Total | 100.0% |

| | |
|---|---|
| *2: Sodium salt of a mixed fatty acid composed of beef tallow/coconut oil = 80/20 (by weight); water content: 13%. | |

The solid body soap thus obtained had excellent detergency and foamability, produced foams pleasant to the touch, and had a high deodorant effect.

## Claims

1. A detergent composition comprising the following components (A), (B) and (C):
(A) 5-95 wt.% of a higher fatty acid salt represented by the general formula (1):
R¹COOM¹ (1)
wherein R¹ is a linear or branched alkyl or alkenyl group having 7-21 carbon atoms, and M¹ is an alkali metal or alkaline earth metal atom, or an ammonium, alkylammonium or alkanolammonium group;
(B) 0.2-5 wt.% of at least one selected from the group consisting of cationic germicides represented by the following general formulae (2), (3), (4) and (5): 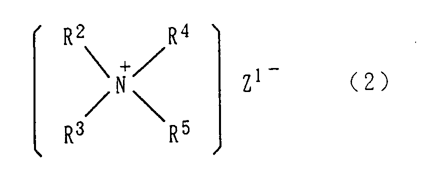 wherein R² and R³ may be the same or different from each other and are independently a long-chain alkyl, long-chain alkenyl or long-chain hydroxyalkyl group having 6-14 carbon atoms, said groups R² and R³ having 16-26 carbon atoms in total, R⁴ and R⁵ may be the same or different from each other and are independently an alkyl or hydroxyalkyl group having 1-3 carbon atoms, or a polyoxyethylene group having an average number of moles of at most 10, and Z¹ is a halogen atom, an anionic residue of an amino acid, a fatty acid, or a phosphate, phosphonate, sulfonate or sulfate having a linear or branched alkyl or alkenyl group having 1-30 carbon atoms, or an anionic oligomer or polymer having a styrenesulfonic acid having a polymerization degree of at least 3 or containing a condensate of a sulfonated polycyclic aromatic compound, which may have a hydrocarbon group as a substituent group, with formalin; 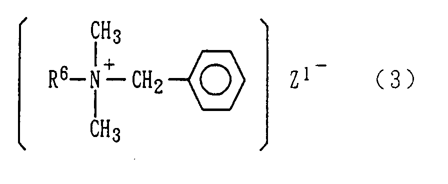 wherein R⁶ is a hydrocarbon group having 8-14 carbon atoms or a group represented by the formula: 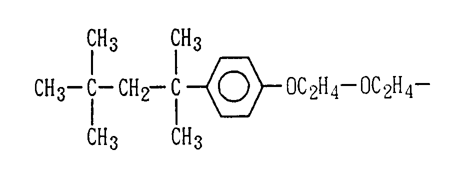 and Z¹ is a halogen atom, an anionic residue of an amino acid, a fatty acid, or a phosphate, phosphonate, sulfonate or sulfate having a linear or branched alkyl or alkenyl group having 1-30 carbon atoms, or an anionic oligomer or polymer having a styrenesulfonic acid having a polymerization degree of at least 3 or containing a condensate of a sulfonated polycyclic aromatic compound, which may have a hydrocarbon group as a substituent group, with formalin; 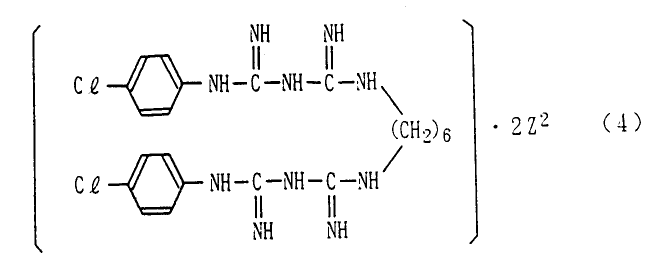 wherein Z² is gluconic acid, acetic acid or hydrochloric acid; and 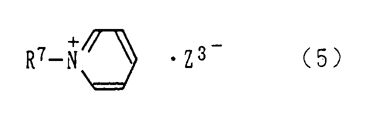 wherein R⁷ is a linear or branched alkyl group having 6-18 carbon atoms, Z³ is a halogen atom, an anionic residue of an amino acid, a fatty acid, or a phosphate, phosphonate, sulfonate or sulfate having a linear or branched alkyl or alkenyl group having 1-30 carbon atoms, or an anionic oligomer or polymer having a styrenesulfonic acid having a polymerization degree of at least 3 or containing a condensate of a sulfonated polycyclic aromatic compound, which may have a hydrocarbon group as a substituent group, with formalin, and polyhexamethylene biguanide; and
(C) 0.1-10 wt.% of at least one selected from the group consisting of amphoteric surfactants, amine oxide type surfactants and amidoamino acid type surfactants, wherein the higher fatty acid salt of the component (A) is contained as a principal detergent base.

2. The detergent composition according to Claim 1, which further comprises, as a component (D), a higher fatty acid represented by the general formula (6):
R¹⁸-COOH (6)
wherein R¹⁸ is a linear or branched alkyl or alkenyl group having 7-21 carbon atoms, in an amount 0.01-1 time as much as the weight of the component (A).

3. The detergent composition according to Claim 1 or 2, wherein the content of the higher fatty acid salt of the component (A) is 5-50 wt.% based on the total weight of the composition, and the composition is in the form of liquid.

4. The detergent composition according to Claim 1 or 2, wherein the content of the higher fatty acid salt of the component (A) is 10-80 wt.% based on the total weight of the composition, and the composition is in the form of paste.

5. The detergent composition according to Claim 1 or 2, wherein the content of the higher fatty acid salt of the component (A) is 60-95 wt.% based on the total weight of the composition, and the composition is in the form of solid.

6. The detergent composition according to any one of Claims 1 to 5, which further comprises at least one metal chelating agent as a component (E).
